# EUROPEAN PATENT APPLICATION

(11) **EP 1 277 835 A1**
(43) Date of publication of application: **22.01.2003**
(21) Application number: 01401944.2
(22) Date of filing: 19.07.2001
(51) Int. Cl.: C12N 15/00, C12N 15/87, C12N 13/00, A01H 1/06, C12Q 1/02, C12Q 1/68

(54) **Methods of creating genetic diversity**

(71) Applicant: Libragen, 69300 Caliure (FR); ECOLE CENTRALE DE LYON, 69131 Ecully Cédex (FR)
(72) Inventor: Nalin, Renaud, 69300 Caliure (FR); Demaneche, Sandrine, 38110 La Tour du pin (FR); Bertolla, Franck, 69100 Villeurbanne (FR); Buret, François, 69130 Ecully (FR); Auriol, Philippe, 69380 Dommartin (FR); Vogel Timothy, 69006 Lyon (FR); Simonet, Pascal, 69100 Villeurbanne (FR)
(74) Representative: Becker, Philippe

(57) **Abstract**

The present invention relates to methods of creating genetic diversity by subjecting an environmental sample to in situ gene shuffling. The invention also relates to methods of making nucleic acid libraries and their uses to screen for biological activities. The invention can be used to create, improve or modify nucleic acid sequences or repertoire, particularly from non-cultivable microorganisms and finds various applications in the medical, food, agriculture or veterinary industries, for instance. The invention uses and discloses in situ gene shuffling.

## Description

The present invention relates to methods for creating genetic diversity. The invention also relates to methods of making nucleic acid libraries and their uses to screen for biological activities. The invention can be used to create, improve or modify nucleic acid sequences, particularly from non-cultivable microorganisms and has various applications in the medical, food, agriculture or veterinary industries, for instance.

Environmental microorganisms represent a rich and unexploited resource of novel compounds and activities. The concept of cloning nucleic acid fragments from environmental samples and creating and screening nucleic acid libraries from these samples has been suggested. Various strategies have been proposed to that effect, which involve isolating nucleic acids from environmental samples, cloning the same in expression vectors to produce libraries, and screening said libraries for the presence of any selected activity. For example, WO98/17811 and WO96/34112 refer to the use of shuttle vectors that replicate in different host strains and contain a promoter region in order to produce such expression nucleic acid libraries. WO99/45154 relates to a method of producing normalized genomic libraries from an environmental sample wherein a nucleic acid amplification step and a normalization step are performed. The libraries are used to screen for various activities, such as enzymes, biosynthesis genes, etc (WO99/10539).

These prior art methods suffer from various drawbacks, such as the limited amount of material contained in the sample, the non-cultivable character of most microorganisms contained in said sample, etc.

The present invention now proposes novel compositions and methods to generate and use genetic diversity. The present invention also relates to novel nucleic acid libraries having expanded genetic diversity, and to novel methods of creating, modifying or improving biological activities or nucleic acid molecules.

The present invention indeed proposes, for the first time, to create genetic diversity by genetic shuffling of microorganisms contained in environmental samples. The present invention more particularly stems from the unexpected discovery that microorganisms contained in environmental samples may be subjected to genetic shuffling *in situ,* thereby creating novel gene sequences, novel protein functions or novel metabolic pathways in said microorganisms. As a result of this *in situ* shuffling, particularly with non-cultivable microorganisms, new sources of compounds, activities or methods are provided, which can be used in various applications.

An object of the present invention thus resides in a method of producing genetic diversity, the method comprising :
a) Providing an environmental sample comprising a plurality of species of (non-cultivable and cultivable) microorganisms, and
b) Subjecting said sample, or a portion thereof, to a gene shuffling step *in situ.*

A further object of this invention is a method of modifying the genetic content of microorganisms, the method comprising :
a) Providing an environmental sample comprising a plurality of species of (non-cultivable and cultivable) microorganisms, and
b) Subjecting said sample, or a portion thereof, to a gene shuffling step *in situ.*

An other object of this invention is a method of making a nucleic acid library , the method comprising:
a) Providing an environmental sample comprising a plurality of species of microorganisms,
b) Subjecting said sample, or a portion thereof, to a gene shuffling step *in situ*,
c) Isolating nucleic acids from said sample, and
d) introducing said nucleic acids into cloning or expression vectors to obtain said library.

Still a further aspect of this invention is a method of making a library of nucleic acids, the method comprising introducing a plurality of nucleic acid fragments into a cloning or expression vector, wherein the plurality of nucleic acid fragments is obtained from a plurality of species of donor microorganisms which have been subjected to a gene shuffling step *in situ.*

The invention also relates to libraries of nucleic acids, wherein said libraries comprise a plurality of cloning or expression vectors comprising a nucleic acid fragment derived from a plurality of species of donor microorganisms which have been subjected to a gene shuffling step *in situ.*

A further aspect of this invention is a library of (or a composition comprising) a plurality of microorganisms which have been genetically altered by *in situ* gene shuffling. Such composition, library or microorganisms have the potential to exhibit new characteristics and represent a source of genetic diversity. Another object of this invention is a library of recipient strains (genetically modified or not) used to recover specific genetic motives by homologous or heterologous recombination(s).

An other object of this invention lies in a method for creating, modifying, improving or identifying a desired biological activity, comprising:
a) Providing a sample comprising a plurality of species of donor microorganisms,
b) Subjecting said sample, or a portion thereof, to a gene shuffling step *in situ,*
c) Isolating nucleic acids from said sample,
d) introducing said nucleic acids into cloning or expression vectors to obtain a library, and
e) screening said library for said desired biological activity.

An other object of this invention is a method for creating, improving, modifying or identifying a nucleic acid encoding a desired biological activity, comprising:
a) Providing a sample comprising a plurality of species of donor microorganisms,
b) Subjecting said sample, or a portion thereof, to a gene shuffling step *in situ,*
c) Isolating nucleic acids from said sample,
d) introducing said nucleic acids into cloning or expression vectors to obtain a library, and
e) screening said library for a nucleic acid encoding said desired biological activity.

The present invention may be used in various areas, such as in the medical, food, agricultural or veterinary industries, for example. The invention can be used to create, modify, improve or isolate biological pathways, including genes or polypeptides involved in the biosynthesis of metabolites (antibiotics, amino acids, pigments, etc.) or in the degradation of chemical compounds, etc.

### Gene Shuffling in situ

The present invention proposes to create genetic diversity by *in situ* genetic (or gene) shuffling of microorganisms contained in environmental samples. The present invention indeed unexpectedly shows that microorganisms contained in environmental samples may be subjected to genetic shuffling *in situ,* thereby creating novel gene sequences, novel protein functions or novel metabolic pathways in said microorganisms. The advantage is to reach and use the enormous biodiversity of untapped microorganisms (non-cultivable) to mix and generate genetic diversity.

"In situ gene (or genetic) shuffling" designates any method of treating microorganisms contained in a sample to stimulate gene shuffling, i.e., the exchange (and/or reorganization) of genetic material. "In situ" indicates that the shuffling is performed on a complex mixture of microorganisms that have not yet been cloned or characterized individually. The complex sample may also be enriched from environmental samples, e.g., through initial pre-treatment.

I*n situ* gene shuffling includes the exchange of genetic material between microorganisms present in the sample, as well as between said microorganisms and free nucleic acids that are also present in the sample. Furthermore, if desired exogenous genetic material or microorganisms may be added to the sample to further increase the shuffling and the resulting diversity.

Gene shuffling may be accomplished through various techniques. In particular, gene shuffling may be chemically or physically induced. However, the present invention now discloses a method that allows an efficient *in situ* shuffling, based on electrical fields and current (electrotransformation), particularly by lightning-mediated (simulated) current injection.

Electrical field and current can permeabilize bacterial membranes allowing for the penetration of naked DNA. Given that the environment is subjected to regular thunderstorms and lightning discharges that induce enormous electrical perturbations, the possibility of natural electro-transformation of bacteria was investigated. We demonstrated with soil microcosm experiments that the transformation of added bacteria could be increased locally via lightning-mediated current injection. The incorporation of exogenous genes encoding three antibiotic resistance (plasmid pBR328) into the *Escherichia coli* strain DH10B recipient previously added to soil was observed after the soil had been submitted to laboratory-scale lightning. Laboratory-scale lightning had similar electrical field gradient (700 vs 600 kV m⁻¹) and current density (2.5 vs 12.6 kA m⁻²) as full-scale lightning. Controls handled identically except for not being subjected to lightning produced no detectable antibiotic resistant clones. In addition, simulated storm cloud electrical fields (in absence of current) did not produce detectable clones (transformation detection limit of 10⁻⁹). Electro-transformation is thus a powerful mechanism to generate diversity in environmental samples.

In a most preferred embodiment of the present invention, *in situ* gene shuffling is thus performed by electrotransformation or lightning-mediated current injection. The electrical parameters of the electrotransformation can be adjusted by the skilled person, depending on the size and nature of the sample. Typically, the current impulse presents an exponential decay in the range of a few microseconds. The peak value of the current depends on the surface of the sample to be treated. The value of the current density can vary from various ranges and depends on the nature of the sample (*ie* bacteria type, soil resistivity). Generally, an electrical field gradient between 100 and 1000 kV m⁻¹ and a current density of between 1 and 20 kA m⁻² may be applied on the sample. Particular conditions use for instance an electrical field gradient of about 700 kV m⁻¹ and current density of about 2.5 kA m⁻². Any commercially available generator may be used to produce electrotransformation, such as the impulse generator of 100Kv - 50 Kj (Haefely - Eserie™). Other suitable conditions are described, for instance, in Rhouma et al (Ninth International Symposium on High Voltage Engineering, August 28-September 1, 1995, Graz Convention Center, Austria, Europe, p. 6730-1), incorporated therein by reference.

As indicated above, the *in situ* gene shuffling can be performed in the presence of exogenous nucleic acids, such as without limitation, genes, gene clusters, biosynthetic pathways, metagenome(s) or microorganisms added to the sample. The addition of such exogenous material further increases the potential genetic diversity. Furthermore, this exogenous material may be added to direct the diversity towards desired properties or features. For instance, by adding cloned exogenous genes involved in a particular pathway (e.g., a biosynthesis pathway), it is possible to favor the creation of novel biosynthesis genes upon shuffling with endogenous chromosomes, or to favor the modification or improvement of the encoded product.

In a particular embodiment, genetic material such as isolated or cloned nucleic acids is added to the sample, prior to shuffling. The isolated or cloned nucleic acids may be naked or incorporated in a vector. These nucleic acids may code for desired polypeptide functions. In particular, it is possible to add clusters of genes encoding a biosynthesis pathway, or a portion thereof, for instance. Particular examples of added exogenous genetic material includes amino acids biosynthesis gene(s), antibiotic biosynthesis gene(s), vitamins biosynthesis gene(s), pigments biosynthesis gene(s), metabolites biodegradation gene(s), insecticide biosynthesis gene(s) or biodegradation gene(s), genes coding for enzymes, etc.

In an other embodiment, whole or partial nucleic acid libraries are added to the sample.

In a further embodiment, whole microorganisms or preparations derived therefrom are added to the sample. The microorganisms may be bacteria, yeast cells, plant cells, insect cells, viruses, animal or human cells, or combinations thereof. Preparations derived from such microorganisms may be used as well, such as a lysate, an RNA preparation, etc.

In situ gene shuffling can be performed in various devices, such as dishes, plates, tubes, flasks, and the like. Typically, the sample is exposed to electrical fields and current in a dish, such as a petri dish.

### Environmental Sample

The term environmental sample designates, broadly, any sample containing (a plurality of) uncharacterized (micro)organisms, particularly uncultivated (or non-cultivable) microorganisms. The sample may be obtained or derived from natural environments or from artificial or specifically created environments (e.g., industrial effluents, etc). An uncultivated (or non-cultivable) microorganism is a microorganism that has not been purposely cultured and expanded in isolated form. Since less than about 1% of the microorganisms or microbes found in nature can be or have been cultured in the laboratory, the advantage of the present invention is to explore and exploit the genetic diversity of all, uncharacterized or uncultivated microorganisms (or microbes).

As indicated, the sample may be any environmental sample such as those obtained or derived from soil, water, mud, vegetal extract, wood, biological material, marine or estuarine sediment, industrial effluents, gas, mineral extracts, sand, natural excrements, etc. The sample may be collected from various regions or conditions, such as tropical regions, deserts, volcanic regions, forests, farms, industrial areas, household, etc.

The sample usually contains various species of (uncharacterized, uncultivated) microorganisms, such as terrestrial microorganisms, marine microorganisms, freshwater microorganisms, etc. Species of such environmental microorganisms include Eubacteria, archaebacteria, algae, protozoa, fungi, viruses, phages, parasites, etc. The microorganisms may include extremophile organisms, such as thermophiles, psychrophiles, psychrotophes, acidophiles, halophiles, etc. More specific examples of environmental bacteria includes actinomycetes, eubacteriaes and mycobacteriaes, examples of fungi include phycomycetes, ascomycetes and basidiomycetes, etc. Other organisms include yeasts (saccharomyces, kluyveromyces, etc.) plant cells (algae, lichens, etc), corals, etc. for instance. The sample may comprise various species of such donor (uncultivated) microorganisms, as well as various amounts thereof. It is indeed believed that the present invention can be performed without precise knowledge of the exact composition of the sample. Furthermore, the sample may contain, in addition, known and/or cultivable microorganisms (e.g., prokaryotic or eukaryotic).

The sample also usually comprises nucleic acids, and organic materials. The sample may also contain mammalian cells or animal cells or insect cells (arising from larvae, feces, etc.).

It should be understood that the present invention is not limited to any specific type of sample or environmental microorganism, but can be used to produce diversity, create nucleic acid libraries, etc., from any environmental sample comprising uncultivated microorganisms.

The sample may be wet, soluble, dry, in the form of a suspension, paste, powder, solid, etc. Preferably, the sample is dry or in solid or semi-solid state (e.g., paste, powder, mud, gel, etc.). The sample may be treated, prior to or after gene shuffling, for instance to enrich for microorganisms. Examples of such treatments include washings, filtrating, centrifuging, diluting, drying, etc. Furthermore, as indicated above, exogenous genetic material may be added to the sample prior to gene shuffling.

### Nucleic Acid Isolation

The present invention discloses methods of creating genetic diversity or nucleic acids or libraries from environmental samples. The methods comprise a gene shuffling step in situ, which increases horizontal gene tranfer, favors or accelerates evolution, and produces novel organisms, novel genetic information and novel resources of products and activities. In situ gene shuffling is much more powerful than in vitro genetic rearrangements since the diversity is much higher either by the diversity of the exogenous nucleic acid which can be introduce or by the diversity of recipient (e.g., donor) strains.

In order to produce nucleic acid libraries or to isolate novel genetic information, the nucleic acids are isolated from the treated sample. Isolation can be carried out according to various techniques, such as those described in PCT/FR01/00922, in WO01/40497, in Handelsman et al (Chemistry & Biology 5(10), 1998, R245), Rondon et al (Tibtech 17, 1999, 403 ; Applied and Environm. Microbiol. 66, 2000, 2541), Miller et al (Applied and Environm. Microbiol. 65, 1999, 4715) or Frostegard et al (Applied and Environm. Microbiol., 65, 1999, 5409).

Isolation typically comprises an extraction and a purification step, which are well known per se in the art. The extraction step aims at releasing the nucleic acid from all or part of the organisms contained in the samples. The purification aims at separating the nucleic acids from other material contained in the sample. Furthermore, during the extraction and/or purification steps, the nucleic acids are preferably subjected to a fragmentation step in order to produce nucleic acid fragments. The nucleic acids may also be subjected to various additional steps or treatments, such as (i) a selection step, in order to purify or retain only nucleic acids having certain physico-chemical or biological properties and to eliminate the others, and/or (ii) an amplification, to expand the nucleic acid population, and/or (iii) a normalization step, etc.

The extraction of nucleic acids can be carried out by chemical, physical and/or enzymatic treatment or lysis, such as, for instance, as described in US5,824,485, WO97/12991, EP939 118 or PCT/FR01/00922. These methods employ detergents, solvents, physical or mechanical treatments, etc. of the sample (or a portion thereof), to release nucleic acids contained in the organisms. The extraction can be direct, i.e., by direct lysis of the organisms in the environmental sample, upon in situ gene shuffling, or indirect, after pre-purification of the organisms, for instance by centrifugation or density (gradient) separation. The latter method allows the production of larger nucleic acid fragments, in the range of 300 kb or more. Indirect nucleic acid extraction is preferred.

Nucleic acid purification can be carried out by various methods known in the art, such as cesium chloride gradient centrifugation, electrophoresis, etc. A preferred method of purifying the nucleic acids is electrophoresis, particularly pulsed field electrophoresis.

The amplification, selection, normalization, etc. may be accomplished as described, for instance, in WO99/45154, WO99/62847, WO96/34112

In a preferred embodiment, the nucleic acid isolation comprises:
1. grinding the sample (or a portion thereof)
2. separating the organisms by sedimentation from the sample obtained in a)
3. extracting the nucleic acids from said separated organisms by lysis, and
4. purifying the nucleic acids by electrophoresis, in particular by pulsed field electrophoresis.

The isolated nucleic acid (fragments) may be genomic DNA, cDNA, RNA, plasmid DNA, mitochondrial DNA or a combination thereof. Preferably, genomic DNA or cDNA is prepared.

The isolated nucleic acids (or fragments) may be pooled to create nucleic acid libraries. To that effect, the nucleic acids are usually inserted, by ligation, into suitable cloning and/or expression vectors. A cloning vector is any vector that allows replication of the cloned nucleic acid fragment in an appropriate host cell. An expression vector is any vector that allows expression (and also, optionally, replication) of the cloned nucleic acid fragment in an appropriate host cell. The expression vector usually contains a regulatory sequence causing expression of the cloned nucleic acid fragment. The regulatory sequence may be any promoter, enhancer, polyA, etc., of cellular, viral or synthetic origin. The cloning and expression vectors may comprise a plasmid, virus, phage, artificial chromosomes (BACs, YACs, , etc.), etc. Typical examples include commercially available plasmids (pUC, pBR, pcDNA, etc.), phages and artificial chromosomes.

In a preferred embodiment, the nucleic acid fragments, or the vectors, are contained in a host cells. The host cells may be any type of cell suitable for culture. These include prokaryotic or eukaryotic cells, such as bacteria, yeasts cells, plant cells, insect cells, mammalian cells, etc. Typical examples of host cells include E.coli, saccharomyces, kluyveromyces, mammalian cells, streptomyces, actinomycetes, etc.

The invention particularly relates to libraries of nucleic acids, wherein said libraries comprise a plurality of cloning or expression vectors comprising a nucleic acid fragment derived from a plurality of species of environmental microorganisms which have been subjected to a gene shuffling step *in situ*.

The invention also relates to libraries of nucleic acids, wherein said libraries comprise a plurality of host cells comprising a cloning or expression vector, said vector comprising a nucleic acid fragment derived from a plurality of species of environmental microorganisms which have been subjected to a gene shuffling step in situ.

The libraries may comprise selected or amplified or normalized nucleic acid fragments.

The libraries thus comprise a pool of cloning or expression vectors, preferably contained in suitable host cells, which represent a genetic diversity. The library usually contains a plurality of cloned nucleic acid fragments having a length ranging between about 1 kb and 600 kb, preferably between 10 and 600 kb. The nucleic acid libraries represent metagenomic DNA from a mixed, uncharacterized population of *in situ*-shuffled organisms.

The libraries may be stocked in any appropriate device, such as dishes, plates, flasks, etc. Replicates may be prepared according to conventional techniques.

The invention also relates to a kit comprising a nucleic acid library as disclosed above. The kit may further comprises reagents or instructions to screen the library.

The libraries can be used to screen for various products or activities. In particular, the invention concerns methods of creating, modifying, improving or identifying a desired biological activity or a nucleic acid encoding a desired biological activity, comprising:
a) Providing an environmental sample comprising a plurality of species of microorganisms,
b) Subjecting said sample, or a portion thereof, to a gene shuffling step in situ,
c) Isolating nucleic acids from said sample,
d) introducing said nucleic acids into cloning or expression vectors to obtain a library, and
e) screening said library for said desired biological activity or for a nucleic acid encoding said desired biological activity.

The screening can be phenotypic, or genetic, for instance. Phenotypic screening would be based on the expression of the cloned nucleic acid fragments in a suitable test host cell or system, and the detection of the presence of a desired biological activity in said test host cell or system. Genetic screening does not require expression, but comprises an initial selection of cloned nucleic acid fragments based on the presence of certain sequence motifs or similarity to a desired target nucleic acid.

The present invention may be used in various areas, such as in the medical, food, agricultural or veterinary industries, for instance. The invention can be used to create, modify, improve or isolate biological pathways, including genes or polypeptides involved in the biosynthesis of metabolites (antibiotics, amino acids, pigments, insecticides, pesticides, secondary metabolites etc.) or in the degradation of chemical compounds, etc. This invention aims at creating new generation of nucleic acid libraries comprising nucleic acids which have been submitted to *in situ* gene shuffling. Such nucleic acid libraries contain shuffled metabolic pathways or genes and can be screened for numbers of applications in different market areas, such as for the discovery of new bioprocess or secondary metabolites (anti-infectious, anticancer, pesticides, insecticides, pigments, adjuvants, polypeptides, enzymes etc...)

Further aspects and advantages of the instant invention will be disclosed in the following examples, which should be regarded as illustrative and not limiting. All references (patent applications, patents, scientific publications) cited in this application are incorporated therein by reference.

### MATERIALS AND METHODS

In situ gene shuffling was performed in soil microcosms seeded with bacteria and plasmids. The laboratory-scale lightning system first simulated charge build up in clouds and then the lightning itself. Afterwards, selective media was used in order to count those bacteria which had acquired the antibiotic resistance encoded on the plasmid. All of these components are described below. In addition, some comparison was made with electroporation in the presence and absence of soil.

**Soil microcosms.** Microcosms consisted of 50 mm Petri dishes in which were placed 30 g (soil thickness :10 mm) of air dried and 2 mm sieved samples of a sandy loam soil (sand, 50%; silt, 41%; clay, 9%; organic matter, 40.6 g. kg of dry soil⁻¹; pH 6.8) collected at La Côte Saint André (Isère, France). Petri dishes were adapted to conduct electricity by sticking a steel wire through a hole in the bottom of the Petri dish and then placing a round piece of aluminum paper inside the Petri dish on the bottom. Thus, the wire went from the aluminum foil at the bottom of the Petri dish, through the hole in the dish bottom and out to where it could be used as an electrical ground in the simulated lightning experiments. Sterile soil conditions were obtained by gamma-irradiating the microcosms by a 25 kGy dose from a ⁶⁰Co source (Conservatome, Dagneux, France). The moisture content for cloud and lightning simulation tests was 10% w/w. The target moisture was achieved in part during the bacterial and plasmid inoculation (2.5 ml per 30 g dried soil) and in part by adding sterile deionized water (0.5 ml per 30 g soil). Soil pH after inoculation and immediately before being subjected to simulated lightning was maintained at 6.8. The soil samples were inoculated with 2.5 ml of the bacterial suspensions and mixed with a sterile pipette. Bacterial suspensions were made based on required final bacterial concentrations in the soil microcosms, which were 10⁷, 10⁸, 10⁹, and 10¹⁰ cells g of dry soil⁻¹. Each bacterial concentration was tested at least 3 times. The seeded soil samples were incubated at 28°C for 18 h before 100 µl of a 10 µg ml⁻¹ plasmid pBR328 (encoding for resistance to ampicillin, chloramphenicol, and tetracycline) solution was added to the soil just prior to the lightning treatment. Controls included (i) soil with the added bacteria, but without the plasmid, (ii) soil with both bacteria and plasmid added, but not subjected to lightning, and (iii) soil with nothing added. Both sterile and non-sterile soil were tested.

**Inoculum and plasmid preparation.** The inoculum of the *Escherichia coli* strain DH10B (Life Technologies, Cergy, France) was prepared by picking a colony maintained on agar and incubating it in 100 ml liquid medium (LB medium) overnight, then 2 ml of the culture was placed in 500 ml LB medium and incubated for about 8 h. When the optical density (at 580 nm) of the liquid culture reached 1, then the liquid was centrifuged for 15 min at 6500 X g before resuspension in the appropriate volume of NaCl 0.8% in order to achieve the targeted final concentration in soil. Confirmation that the *Escherichia coli* strain DH10B could not be naturally transformed in vitro was done by mixing 40 µl of plasmid pBR328 DNA (Boehringer Mannheim, Meylan, France) and recipient cells on GTTP filters (Millipore, Ireland) on solid LB medium. After incubation for 24 h at 37°C filters were resuspended in 2 ml of pure sterile water and suspensions plated on selective media (LB medium containing, per liter, ampicillin, 100 mg; chloramphenicol, 30 mg; tetracycline, 12.5 mg). The plasmid pBR328 was isolated from *E. coli* by using the plasmid extraction kit from Qiagen Inc. (Chatsworth, California).

**Simulation of cloud induced electrical field.** When only the electrical field associated with clouds was tested without lightning, conditions corresponding to natural cloud induced electrical fields were simulated by subjecting seeded soil samples to a static electrical field created through a large planar capacitor structure, the distance between electrodes was 0.25 m. The apparatus was capable of delivering DC voltage values ranging from 4 to 140 kV. In these experiments, when the 140 kV (V crest) was used over the 250 mm between electrodes, the gradient (Ecrest) was about 0.56 kV mm⁻¹, which is similar to that of storm clouds (about 0.6 kV mm⁻¹) that produce lightning (Table 1). Soil samples were exposed to this field for different periods (1, 5, 10 and 60 min).

**Lightning simulation**. The experimental system was based on a high voltage generator which could deliver impulses up to 1 MV with 50 kJ energy. This generator was constructed in order to test high voltage apparatus performance and the ability of different electronic equipment to survive lightning hits (3). For the soil application, the current flowed from an electrode through the soil to the aluminum foil at the bottom of the Petri dish, which was directly sitting on a solid bronze disk (0.5 m in diameter and 2 cm thick) which itself was sufficiently grounded for the applied current. The electrode was a cylindrical cell (bronze, 2.5 cm diameter and 3.5 cm high) that was capable of applying voltage impulses up to 8.9 kV with a time constant value equal to 6 ms. Under these conditions the current flowing through the soil can reach more than 6 A. For typical natural lightning the duration of the current impulse of each return stroke can vary significantly from 0.1 to 1 ms (20).

**Detection of transformants.** Following the lightning discharge, a subsample of soil (approximately 2 g) was recovered and treated with DNase I (Boehringer, Mannheim, Germany) to degrade any persistent extracellular DNA (incubation for 2 h at 28°C with 1000 U. of DNase I). Detection protocols included either (i) quantitative analysis by direct plating of part of this soil sub-sample (and dilutions of this subsample with the selective media) on selective media (LB medium containing, per liter, ampicillin, 100 mg; chloramphenicol, 30 mg; tetracycline, 12.5 mg and in order to inhibit fungal growth, amphotericin B, 1.25 mg) to enumerate transformants or (ii) qualitative analysis by incubating the whole 30 g of soil in the microcosm overnight at 28°C in 20 ml of selective media before plating 5 X 100 µl on selective media. Although this last technique did not allow exact counting of transformants, it increased detection sensitivity as any transformants could develop over the incubation period, therefore, its theoretical detection limit was one transformant per 30 g of soil. Any apparent transformants were confirmed to contain the added plasmid by using a plasmid extraction kit (Qiagen Inc., Chatsworth, California) according to manufacturer instructions. For experiments with sterile soils, recipient bacteria (both those which had incorporated the plasmid and those that had not) were enumerated on LB media.

**Electroporation.** Electrocompetent cells of *Escherichia coli* strain DH10B were prepared as described by Drury (8). Electroporations were carried out with the gene pulser ™ apparatus (Bio-Rad Laboratories, Richmond, CA, USA) by using 40 µl of recipient cells mixed with 1 µl of plasmid pBR328 solution (100 mg L⁻¹), incubated 1 min on ice and electroporated in 0.2 cm cuvette at 1.2 kV mm⁻¹ (crest value) during 4 to 5 ms. LB medium was then immediately added (960 µl) and the suspension incubated for 1 h at 37°C before the dilutions were plated on both LB media alone and LB media with antibiotics (as described above) to estimate both survival and transformation frequencies, respectively. In addition, in order to try to incorporate the soil aspect in the electroporation tests, 75 mg of sterile soil was placed in the electroporation cuvette, then 41 µl of the bacteria-plasmid suspension was added, then an additional 75 mg of soil was added before performing the electroporation.
To simulate our "cloud" experiments (where only an electrical field and no current is applied) with the electroporator , we used specifically a 0.45 cm wide disposable Plastibrand cuvettes on which electrodes were added to the outside (hence not in contact with the solution), thus with the application of voltage in the electroporator, no current occurred, but an electrical field was established. This was only tested on bacterial plasmid solutions in the absence of soil.

**Comparison of electrical parameters between electroporation and lightning current propagation in soils.** Three electrical parameters were used to compare the two laboratory-scale systems (electroporation and simulated lightning) and full-scale lightning (Table 1). The first parameter was the crest value of the electrical field (E_{crest}) which is obtained for natural conditions by dividing the maximum electrical tension value reached during the electrical impulse by the electrode distance (V_{crest}/d) or for natural lightning by multiplying the maximum value of the current density by the electrical resistivity of the soil tested (J_{crest} X Ω). The second parameter was the crest value of the current density (J_{crest}) which corresponds to the maximal current intensity divided by the cell cross section (I_{crest}/s) which provides data that can compare systems of different size-scale. The third parameter was the amount of electrical charge flowing through the cell for a 1 m² surface (∫_{j(t)dt}). The laboratory conditions simulated very closely those occurring in soil during a lightning discharge. The crest values of the electrical field (E_{crest}) were nearly identical for natural and artificial lightning and differed by less than a factor 2 for electroporation. The crest value of the current density (J_{crest}) and the amount of charge flowing through the soil were similar for the three different conditions in a range from 4 to 22 C m⁻² although the surface and volume of soil for natural lightning could be determined only approximately. The variations in Table 1 are not significant with respect to our hypothesis when one realizes that during the lightning event, considerable variation in these values occur over the soil area hit by lightning (2, 20).

### EXAMPLE 1 - Electric Gene shuffling

**Thunderstorm related electrical parameters and potential biological effects.** Simulated storm cloud electrical fields subjected soil seeded with a recipient bacteria, *Escherichia coli* strain DH10B, and donor DNA, plasmid pBR328, to static electrical fields as described above. These experiments failed to provide any detectable transformant whatever the various conditions tested, which included a range of recipient bacteria concentrations (from 10⁷ to 10¹⁰ cells g of dry soil⁻¹), donor DNA concentrations (from 1 to 100 µg plasmid pBR328 g of dry soil⁻¹), and ranges in the generated electrical field amplitude (from 0.16 to 0.6 kV mm⁻¹) and duration (1 to 60 min). These results are consistent with the lack of transformants in vitro when the electroporation cuvette was modified to subject bacteria only to electrical fields, but without current. Efficiencies of transformation of *Escherichia coli* strain DH10B (expressed as the number of transformants relative to that of recipient cells) by 0.5 µg of plasmid pBR328 DNA dropped from 1.3 x 10⁻⁵ with standard electroporation conditions to less than 7 x 10⁻⁹ (the detection limit) with modified ones indicating the requirement of electrical current for DNA transfer. These results imply that the accumulation of clouds alone (electrical field without current) might not have any significant effect on HGT.

**Artificial lightning related electro-transformation of bacteria in soil.** Experiments were developed in order to subject soil samples seeded with recipient bacteria and donor DNA to laboratory-scale lightning delivered by a high tension generator. The use of a non-naturally transformable bacterium such as *Escherichia coli* DH10B and a plasmid harboring three antibiotic resistance marker genes (pBR328) allowed for the easy detection of transformants.
Transformants were detected in all samples containing from 10⁵ to 10⁹ recoverable bacteria cells g of dry soil⁻¹ when plasmid concentration was greater than 0.1 µg DNA g of dry soil⁻¹ (Table 2). When plasmid concentration was 0.1 µg DNA g of dry soil⁻¹ transformants were detected qualitatively, but rarely of sufficient number to quantify the transformation frequency (Table 2). The qualitative method appeared to be quite sensitive to the presence of transformants, thus, increasing the certainty that "no lightning" controls did not produce any transformants (Table 2). Controls with lightning but without the plasmid were also absent of any detectable transformants. When non-sterile soils were tested, the *Escherichia coli* DH10B was unable to maintain its population at elevated levels and, therefore, the actual target bacteria population was not identified and no clones were observed using the quantitative method, although the qualitative method did provide evidence of transformants.

**Estimation of the HGT level in soils submitted to natural lightning.** These experimental results indicate that bacteria in soils are affected by lightning related electrical parameters, which, like those delivered by a standard electroporator, increase their permeability to extracellular DNA. Soils in our microcosms were homogenized in order to have the current delivered homogeneously all over the soil sample. On the other hand, the current delivered by lightning discharges is going to flow along channels of reduced resistivity. These channels are characterized by very high current densities and a considerable increase of the temperature possibly submitting bacteria to irreversible damages and death. Nevertheless, magnitude of the electrical parameters (current density, electric field) decreases with distance from the axis of these conducting channels creating electrical and thermic conditions compatible with bacterial survival and potentially with electro-transformation in a large volume of the soil surrounding these "over-stressed" areas. Therefore most lightning discharges would provide transformation-inducing electrical conditions at some places in soil independent of its structure, texture and matrix characteristics similar to that described here for the laboratory-scale lightning.
Another critical aspect of lightning-induced gene transfers in nature is the state of natural extracellular donor DNA. Significant amounts of indigenous extracellular DNA in a range from 5 to more than 35 µg g of dry soil⁻¹ depending upon the soil composition can be routinely detected and even extracted (9). There are however, few data on the transformation potential of these natural molecules. Even when a significant part of DNA inoculated into non sterile soils is rapidly degraded, another fraction could persist by adsorption onto soil particles and remain available to transformation mechanisms (5, 19). Moreover, the closed micro-habitats due to soil aggregation (17) in which most indigenous soil bacteria develop, die and release DNA might be much more favorable to natural electro-transformation than outside the soil aggregate. The electrophoretic forces that are hypothesized to drive DNA into the transforming cells (12, 13) might be involved in the desorption of DNA molecules from soil particles (15) contributing to their increased availability. Also, intracellular DNA present in living, dying, and dead cells could be a major source of donor DNA in addition to extracellular DNA based on evidence that electroporation was efficient in vitro to generate protoplast fusion (18) and even to transfer DNA between living bacterial cells (10). Finally, given that cells from nearly all bacterial taxa can be electroporated more or less efficiently and independently of their physiological state and with transforming DNA of prokaryotic or eukaryotic origin, the universality of this process needs to be considered.
Based on these various experimental and theoretical data, frequency of natural lightning-mediated gene transfers in soils might not be vastly different from a value of 10⁻⁹ resulting from our simulation experiments (Table 2). Natural lightning discharges spread over 0.5 to 4 m³ of soil containing more than 10¹² to 10¹³ indigenous bacterial cells potentially affected by the electrical parameters, thus providing a theoretical number of transformants up to 10⁴ at each discharge. The hundreds of lightning flashes subjecting the environment to thousands of coulombs each second on a world-wide-scale could be participating in bacterial adaptation and evolution.

### EXAMPLE 2 - Production of Nucleic Acid Libraries

**Sample Preparation and shuffling.** Environmental samples are collected from soils, sediments, water, waste, animals, insects etc. The sample is subjected to gene shuffling as described in Example 1.
In the case of complex samples such as soil, it is important to sieve and homogenize the sample in order to have access to the maximal biodiversity. Foe example, 5g to 10 Kg of soil samples have to be sieved (2mm mesh sieve). The soil can be resuspended either in physiological or lysis solutions and grinded using different apparatus such as Waring Blender for 3 min at cold temperature.

**DNA extraction and purification.** DNA are isolated either by direct or indirect extraction methods from the above environmental samples.

### Direct extraction methods:

Direct isolation of DNA from environmental samples consists in separating the nucleic acids from all other environmental or cellular particles.

Soil sample is resuspended in lysis solution such as:
- TENP pH 8 (Tris 50 mM, EDTA 100 mM, NaCl 100 mM, polyvinylpolypirrolidone 1% w/v
- Detergents such as CTAB (hexadecyltrimethylammonium) lauryl sarcosyl, SDS (sodium dodecyl sulfate) and preferentially from 1 to 2 %
- Lysosyme (10mg/ml)
- Achromopeptidase (1 mg/ml)
- Proteinase K (2mg/ml)

Environmental particles are separated from the nucleic acid solutions preferentially by centrifugation (7000 g for 15min). Nucleic acids are extracted from the supernatant by standard organic extraction methods, for example (phenol/chloroform/ isoamylic alcohol) followed by one or two chloroform extractions, and precipitated generally with ethanol or isopropanol in the presence of salts (sodium or ammonium acetate).

### Indirect extraction methods:

Indirect nucleic acid extraction methods consist in the separation of the cells (intact cellular fraction) from the environmental samples by density gradient ( such as: percoll, iodixanol, nycodenz, ficoll) before nucleic acid extraction is performed.
For example, 10ml of Nycodenz solution (Nycomed Pharma AS, Oslo, Norvège) with 1.3 density (8g of Nycodenz suspended in 10 ml of sterile water) is deposited under 2.5g of grinded soil sample, suspended in 25ml of physiological solution (NaCl 0.8%) into a centrifugation tube. Centrifugation is carried on at 4°C during 30min at 10 000g in a swing out rotor (TST 2838 Kontron).

The cellular fraction layering on the nycodenz phase is collected after visual observation by direct pipeting. The collected cellular fraction is subsequently washed with 25 ml of ultrapure water and then centrifugated 20 min at 10 000 g in the same rotor.

Pelleted cellular fraction is resuspended in lysis solution as described above and submitted to nucleic acid extraction by standard methods (cell lysis/ organic extraction/alcohol precipitation) or by using commercial nucleic acid extraction kits.

An other method to extract and purify nucleic acids is to use different salt of cesium chloride (such as: cesium chloride, Cesium Trifluoroacetate (CsTFA), iodixanol etc...) density gradient where the nucleic acid can be directly extracted and subsequently purified by centrifugation. The centrifugation can be achieved at 60 000 rpm for 16h at 18°C in rotor (Kontron 80.4) for example.

In a preferred embodiment high molecular weight DNA is obtained from the cellular fraction by embedding in low melting agarose before cell lysis and enzymatic manipulation of DNA to be carried out. Cell lysis in low melting plugs is achieved by incubation of plugs in lysis solutions containing, in a preferred embodiment a TE (50 mM Tris, 100 mM EDTA) complemented with lysosyme 5mg/ml and achromopeptidase 0,5mg/ml (first lysis overnight at 37°C), then twice in a lysis solution containing a TE (50/100) complemented with a detergent at 1% (lauryl sarcosyl, or sodium dodecyl sulfate) and with 0,2 mg/ml proteinase K (second and third overnight incubations at 55°C). Proteinase K has to be inactivated by PMSF action (2h at 4°C, 0.1 mM).
Plugs are rinsed several times in TE (10/1) and incubated for enzymatic restriction as widely described in literature.
Pulse field electrophoresis is run as described in PCT/FR01/00922.

The DNA obtained is pure enough for successive standard molecular methods. Cloning can be done by using cohesive or blunt ligation or by using adaptors or homopolymer tailing as described. Cloning vectors are plasmid, fosmid, cosmid, phage derivative, artificial chromosome (BAC, PAC, YAC, etc.). Depending on the vector used, the transformation is carried out via encapsidation in the case of phage derivative systems, infection in the case of cosmids, electroporation in the case of artificial chromosome and chemical or electroporation in the case of plasmids.

The resulting DNA libraries are stocked in 96 or 384 wells plates at (- 80°C) before further uses thereof for molecular, chemical or phenotypical screening.

### DISCUSSION

Ongoing sequencing and comparing of bacterial genomes are gradually modifying our understanding of how bacteria evolved. Although earlier ideas included a point-mutation-based evolution occurring slowly and regularly, new theories propose a more erratic evolution in which drastic changes in the genome are due to irregular acquisition of new genetic information by horizontal gene transfers (HGT) (14). However, the frequency of HGT would have had to been so high during bacterial evolution that some evolutionists are questioning the accuracy of phylogenetic analyses (6). Moreover, these techniques underestimate the actual number of transfers by missing the oldest events. Their efficiency is also limited to those of the transferred genes, which were successfully fixed in a microbial population by increasing the overall fitness. These sequence-based tools also miss the great majority of transfer events that conferred neutral or deleterious traits and, thus, were subsequently deleted. In addition, numerous microcosm-based investigations devoted to studying these events and specifically natural genetic transformation in environments, such as soil, sediment and water, conclude that HGT would occur at extremely low frequencies around (11, 16).

Natural transformation of bacteria in soil by extracellular DNA is a multi-step process precluding its occurrence at high frequency (16). When released into the soil at the death of organisms (or more actively for some bacteria), naked DNA has to avoid both enzymatic degradation and irreversible adsorption onto soil particles (5). Moreover, when taken up into the cell, transforming DNA still has to resist the numerous restriction and modification mechanisms and to be integrated into the host genome via homologous or illegitimate recombination (or to replicate autonomously). The intermediate step between these extra- and intra-cellular states corresponds to the transformation mechanism sensu stricto. When genetically encoded, this mechanism requires the bacterial cells to be in a competent state, which is another major limitation of transformation in situ (16).

The discrepancy between databases indicating HGT has occurred frequently (14) in the past and the extremely low frequency measured in the environment (11, 16) has led to questions about whether experiments targeted the appropriate bacteria, the important environmental habitats and/or the correct processes. For instance, the soil bacterium *Ralstonia solanacearum*, a plant pathogen, exhibits a potential for HGT several orders of magnitude higher in plant tissues than in bulk soils (4). This is due to the rapid and extensive multiplication of the pathogen leading to the development in situ of the physiological state of genetic competence. As a second example, some transformants of *Escherichia coli,* a bacterium which is not fitted with the appropriate molecular machinery to develop competence were detected in fresh water indicating that transformation can also be physically or chemically induced in situ (1). Therefore, our objective was to evaluate the possibility of other processes being involved in HGT. Considerable data has already been collected concerning the mechanism and efficiency of gene exchange in laboratory devices using electrical fields and currents (7, 10, 12, 21). Thus, given that the environment is regularly subjected to thunderstorms and lightning discharges, our objective was to investigate whether the resulting electrical perturbations could lead to the "natural" transformation of bacteria. Thunderstorm development are processes which include a progressive accumulation of stormy clouds followed by lightning discharges. Electrical parameters related to each of these steps have been investigated extensively and are now well characterized (2). An electrical potential difference builds up gradually with cloud growth. In temperate climates, clouds are positively charged in their upper zone and negatively at the bottom resulting in a modification of the charge and of the electrical field distribution on the ground. The voltage difference between the cloud and the ground can reach up to 100 MV, corresponding to values around 16 kV m⁻¹ (0.016 kV mm⁻¹) for flat ground surfaces, increasing considerably locally due to a tip effect (up to 0.7 kV mm⁻¹) (Table 1). Our experimental objectives were to determine whether these electrical fields related to cloud accumulation could be involved in genetic transformation of bacteria and if not alone perhaps with the addition of electrical current in the form of lightning. Lightning discharges to the soil are characterized by an injection of current with a peak current intensity of the flashes varying from 10 to 200 kA (2), which is considered to flow through about 2 m² of soil surface. On average, this results in about 13 kA m⁻² (Table 1).

The results reported here demonstrate the possibility of in situ electro-transformation of non-cultivable microorganisms by submitting artificially-seeded soil microcosms to electrical perturbations simulating both storm cloud electrical fields and lightning. These results open new avenues for the creation of genetic diversity and the isolation of novel products or activities.

### References

1. **Baur, B., K. Hanselmann, W. Schlimme, and B. Jenni.** 1996. Genetic transformation in freshwater: *Escherichia coli* is able to develop natural competence. Appl. Environ. Microbiol. **62**:3673-3678.
2. **Ben Rhouma, A., and P. Auriol.** 1997. Modelling of the whole electric field changes during a close lightning discharge. J. Phys. D : Appl. Phys. **30**:598-602
3. **Ben Rhouma, A., Auriol, P., and P Dumas** 1995: Laboratory experiments reproducing the nearby lightning electromagnetic fields. 9th Int. Symposium on High Voltage Engineering, Graz (Austria), August 28- September 1, 1995.
4. **Bertolla, F., A. Frostegard, B. Brito, X. Nesme, and P. Simonet.** 1999. During infection of its host, the plant pathogen *Ralstonia solanacearum* naturally develops a state of competence and exchanges genetic material. Mol. Plant-Microbe Interact. **12:**467-472.
5. **Demanèche, S., L. Jocteur-Monrozier, H. Quiquampoix, and P. Simonet.** 2001. Evaluation of biogical and physical protection against nuclease degradation of clay-bound plasmid DNA. Appl. Environ. Microbiol. 67:293-299.
6. Doolittle, W. F. 1999. Phylogenetic classification and the universal tree. Science **284**:2124-2129.
7. **Dower, W. J., J. F. Miller, and C. W. Ragsdale.** 1988. High efficiency transformation of *E. coli* by high voltage electroporation. Nucleic Acids Res. **16**:6127-6145.
8. **Drury, L.** 1996. Transformation of bacteria by electroporation. *In* A. Harwood, (ed.), Methods in molecular biology. Humana Press Inc., Totowa, N.J.
9. **Frostegard, A., S. Courtois, V. Ramisse, S. Clerc, D. Bernillon, F. Le Gall, P. Jeannin, X. Nesme, and P. Simonet.** 1999. Quantification of bias related to the extraction of DNA directly from soils. Appl. Environ. Microbiol. **65**:5409-5420.
10. **Gilchrist, A., and J. Smit.** 1991. Transformation of freshwater and marine caulobacters by electroporation. J. Bacteriol. **173**:921-925.
11. **Lorenz, M. G., and W. Wackernagel.** 1994. Bacterial gene transfer by natural genetic transformation in the environment. Microbiol. Rev. **58:**563-602.
12. **Lurquin, P. F.** 1997. Gene transfer by electroporation. Mol. Biotechnol. **7:**5-35.
13. **Neumann, E.** 1992. Membrane electroporation and direct gene transfer. Bioelectrochem. Bioenerg. **28:**247-267.
14. **Ochman, H., J. G. Lawrence, and E. A. Groisman.** 2000. Lateral gene transfer and the nature of bacterial innovation. Nature **405**:299-304.
15. **Paget, E., L. Jocteur Monrozier, and P. Simonet.** 1992. Adsorption of DNA on clay minerals: Protection against DNase I and influence on gene transfer. FEMS Microbiol. Lett. **97:**31-40.
16. **Paget, E., and P. Simonet.** 1994. On the track of natural transformation in soil. FEMS Microbiol. Ecol. **15**:109-118.
17. **Ranjard, L., F. Poly, J. Combrisson, A. Richaume, and S. Nazaret.** 1998. A single procedure to recover DNA from the surface or inside aggregates and in various size fractions of soil suitable for PCR-based assays of bacterial communities. Eur. J. Soil. Biol. **34**:89-97.
18. **Reed, W. M.** 1987. Protoplast fusion of *Lactobacillus acidophilus* and *Streptococcus lactis* via electric field or chemical induction. J. Gen. Appl. Microbiol. **33**:287-294.
19. **Romanowski, G., M. G. Lorenz, G. Sayler, and W. Wackernagel.** 1992. Persistence of free plasmid DNA in soil monitored by various methods, including a transformation assay. Appl. Environ. Microbiol. **58**:3012-3019.
20. **Uman, M.A.** 1987. : The Lightning Discharge. Int. Geophysics Series, **39**:137, Ed.: W. L. Donn, Academic Press, NY.
21. **Weaver, J. C., and Y. A. Chizmadzhev.** 1996. Theory of electroporation: A review. Bioelectrochem. Bioenerg. **41**:135-160.

**TABLE 1.**

| Comparison of electrical parameters averaged over the current injection period between electroporation, artificial and natural lightning | | | | | |
|---|---|---|---|---|---|
| System | V_{crest} (kV) | E_{crest} (kV mm⁻¹) | I_{crest} (A) | J_{crest} (A m⁻²) | ∫j(t)dt (C m⁻²) |
| Electroporation | 2.4 | 1.2 | 0.1 | 5,000 | 22.5 |
| Simulated lightning | 7.0 | 0.7 | 5.0 | 2,500 | 15 |
| Natural lightning | i^{a} | 0.6^{b} | 26,000 | 12,000 | 4.15 |

| | | | | | |
|---|---|---|---|---|---|
| V_{crest} is the total voltage applied; E_{crest} is the vertical voltage gradient; I_{crest} is the total current and J_{crest} is the average current density over the surface of the soil; ∫j(t)dt corresponds to the amount of charge flowing through the cell for a 2 m² surface. ^{a} i, irrelevant. | | | | | |
| ^{b} This value was calculated for an electrical resistivity of the soil tested of 50 Ω m. | | | | | |

**TABLE 2.**

| Laboratory-scale lightning mediated transformation of *Escherichia coli* strain DH10B by plasmid pBR328 | | | | |
|---|---|---|---|---|
| SYSTEM | CONDITIONS | | | RESULTS |
| Laboratory-scale Lightning (kV / A)^{a} | Plasmid pBR328 concentration^{b} | *Escherichia coli* strain DH10B concentration^{c} | Transformants after 24-h incubation^{d} | Frequency^{e} |
| 7.0/8.1 | 0.4 | 2.3 X 10⁹ | > 10⁴ | 1.1 X 10⁻⁸ |
| | | | | (2.5 X 10⁻⁹) |
| 7.0 / 8.1 | 0.1 | 2.3 X 10⁹ | > 10³ | ND^{f} |
| 5.0 / 6.5 | 0.4 | 3.6 X 10⁸ | > 10⁴ | 1 X 10⁻⁸ |
| | | | | (5.8 X 10⁻⁹) |
| 5.0 / 6.5 | 0.1 | 3.6 X 10⁸ | > 10² | ND |
| 6.0 / 5.2 | 0.4 | 1.2 X 10⁸ | > 10³ | 4 X 10⁻⁹ |
| | | | | (1 X 10⁻⁹) |
| 6.0 / 5.2 | 0.1 | 1.2 X 10⁸ | > 10³ | ND |
| 4.0 / 5.5 | 0.4 | 2.2 X 10⁸ | > 10³ | ND |
| 4.0 / 5.5 | 0.1 | 2.2 X 10⁸ | 0 | ND |
| 5.0 / 4.3 | 0.2 | 3.3 X 10⁸ | > 10⁴ | 1.6 X 10⁻⁷ |
| | | | | (1.3 X 10⁻⁷) |
| 8.0 / 5.8 | 0.2 | 2.6 X 10⁶ | > 10³ | ND |
| 8.0 / 5.8 | 0.2 | 1.0 X 10⁵ | > 10 | ND |
| 7.0 / 4.7 | 0.2 | 1.0 X 10^{9 g} | > 10² | ND |
| | 0.2 | 1.3 X 10⁵ | > 10³ | 2 X 10⁻⁵ |
| | | | | (8 X 10⁻⁶) |
| | 0.2 | 3.3 X 10⁵ | > 10³ | ND |
| Controls | 0.4 | 4.0 X 10⁸ | 0 | ND |
| without | 0.1 | 4.0 X 10⁸ | 0 | ND |
| Lightning^{h} | 0.4 | 2.3 X 10⁸ | 0 | ND |
| | 0.1 | 2.3 X 10⁸ | 0 | ND |
| | 0.2 | 3.3 X 10⁵ | 0 | ND |
| Controls with | 0 | 3.3 X 10⁵ | 0 | ND |
| lightning | 0 | 4.0 X 10⁸ | 0 | ND |

| | | | | |
|---|---|---|---|---|
| ^{a} Electrical field potential (kV) and simulated lightning current (A) applied to soil microcosms | | | | |
| ^{b} Concentrations are in µg DNA g of dry soil⁻¹. | | | | |
| ^{c} Concentrations are in CFU g of dry soil⁻¹. | | | | |
| ^{d} Concentrations are in clones g of dry soil⁻¹. | | | | |
| ^{e} Frequency based on number of clones per total *Escherichia coli* strain DH10B counted in soil with standard deviation based on minimum three measurements in parenthesis. | | | | |
| ^{f} ND, frequency not determined due to lack of clones. | | | | |
| ^{g} This soil sample was not sterilized before the experiment. The quantity of recipient bacteria was not measured. The reported value is the initial inoculated concentration. | | | | |
| ^{h} Controls were identical in every way except for not experiencing laboratory-scale lightning. | | | | |

## Claims

1. A method of producing genetic diversity, the method comprising :
a) Providing an environmental sample comprising a plurality of species of microorganisms, and
b) Subjecting said sample, or a portion thereof, to a gene shuffling step in situ.

2. A method of modifying the genetic content of microorganisms, the method comprising:
a) Providing an environmental sample comprising a plurality of species of non-cultivable microorganisms, and
b) Subjecting said sample, or a portion thereof, to a gene shuffling step in situ.

3. A method of making a library of nucleic acids, the method comprising:
a) Providing an environmental sample comprising a plurality of species of microorganisms,
b) Subjecting said sample, or a portion thereof, to a gene shuffling step in situ,
c) Isolating nucleic acids from said sample, and
d) introducing said nucleic acids into cloning or expression vectors to obtain said library.

4. A method of making a library of nucleic acids, the method comprising introducing a plurality of nucleic acid fragments into a cloning or expression vector, wherein the plurality of nucleic acid fragments is obtained from a plurality of species of donor microorganisms which have been subjected to a gene shuffling step in situ.

5. The method of any one of claims 1 to 4, wherein the gene shuffling step in situ is performed by electrotransformation or lightning-mediated current injection.

6. The method of any one of claims 1 to 5, wherein the gene shuffling step in situ is performed in the presence of exogenous nucleic acids or microorganisms added to the sample.

7. The method of any one of claims 3 to 6, wherein the nucleic acid fragments are genomic DNA, cDNA, plasmid DNA, mitochondrial DNA or a combination thereof.

8. A library of nucleic acids, wherein said library comprises a plurality of cloning or expression vectors comprising a nucleic acid fragment derived from a plurality of species of donor microorganisms which have been subjected to a gene shuffling step in situ.

9. A library of (or a composition comprising) a plurality of uncharacterized microorganisms which have been genetically altered by *in situ* gene shuffling.

10. A method for creating or identifying a desired biological activity, comprising:
a) Providing an environmental sample comprising a plurality of species of donor microorganisms,
b) Subjecting said sample, or a portion thereof, to a gene shuffling step in situ,
c) Isolating nucleic acids from said sample,
d) introducing said nucleic acids into cloning or expression vectors to obtain a library, and
e) screening said library for said desired biological activity.

11. A method for creating, improving, modifying or identifying a nucleic acid encoding a desired biological activity, comprising:
a) Providing an environmental sample comprising a plurality of species of donor microorganisms,
b) Subjecting said sample, or a portion thereof, to a gene shuffling step in situ,
c) Isolating nucleic acids from said sample,
d) introducing said nucleic acids into cloning or expression vectors to obtain a library, and
e) screening said library for a nucleic acid encoding said desired biological activity.

12. The method of claim 10 or 11, wherein the gene shuffling step in situ is performed in the presence of exogenous nucleic acids or microorganisms added to the sample.

13. A kit comprising a library of claim 8 or 9.

14. A nucleic acid encoding a desired biological activity, obtained by a method of claim 11.
